# EUROPÄISCHE PATENTANMELDUNG

(11) **EP 2 158 864 A2**
(43) Veröffentlichungstag der Anmeldung: **03.03.2010**
(21) Anmeldenummer: 09010717.8
(22) Anmeldetag: 20.08.2009
(51) Int. Cl.: A61B 17/80

(54) **Handgelenkarthrodeseplatte sowie Sortiment**

(30) Priorität: 26.08.2008 DE 102008039693
(71) Anmelder: NORMED Medizin-Technik Vertriebs-GmbH, 78532 Tuttlingen (DE)
(72) Erfinder: Pech, Uwe, 78532 Tuttlingen (DE); Wörner, Roland, 78532 Tuttlingen-Möhringen (DE); Neumann, Axel, 80686 München (DE)
(74) Vertreter: Wagner, Kilian

(57) **Zusammenfassung**

Die Anmeldung betrifft eine Handgelenkarthrodeseplatte (1), umfassend einen proximalen Fixierabschnitt (2) zur Fixierung der Handgelenkarthrodeseplatte (1) am Radius (12) sowie einen, mehrere Durchgangslöcher (9) zur Aufnahme jeweils mindestens einer Knochenschraube (14) aufweisenden, Handwurzelknochenfixierabschnitt (3) zum Fixieren der Handgelenkarthrodeseplatte (1) an mindestens einem Handwurzelknochen (11). Es ist vorgesehen, dass die Durchgangslöcher (9) im Handwurzelknochenfixierabschnitt (3) derart quer zur Längserstreckung der Handgelenkarthrodeseplatte (1) beabstandet angeordnet sind, dass in mindestens zwei quer zur Längserstreckung der Handgelenkarthrodeseplatte (1) nebeneinander angeordnete Handwurzelknochen (11) jeweils mindestens eine Knochenschraube (14) einschraubbar ist.

## Beschreibung

### Stand der Technik

Die Erfindung betrifft eine Handgelenkarthrodeseplatte gemäß dem Oberbegriff des Anspruchs 1 sowie ein Sortiment, umfassend mindestens zwei unterschiedliche Handgelenkarthrodeseplatten gemäß Anspruch 13.

Die Handgelenkarthrodese ist ein etabliertes chirurgisches Verfahren, das vorausschaubar Schmerzen lindert und ein stabiles Handgelenk für den Kraftgriff liefert. In der Praxis werden zur Versteifung des Handgelenks dorsale, langgestreckte Knochenplatten benutzt. Diese bieten die Möglichkeit, die Knochenplatte zum einen am Radius (Speiche) und zum anderen an einem Mittelhandknochen zu fixieren. Schraubverankerungen in den Handwurzelknochen sind über zwei, maximal drei Durchgangslöcher möglich, die in Richtung der Längserstreckung der Knochenplatte hintereinander angeordnet sind. Ferner von Nachteil bei bekannten Knochenplatten ist, dass diese, insbesondere im Bereich des Mittelhandknochens, relativ dick auftragen. Hier kann es zu Weichteilstörungen bzw. Wundheilstörungen kommen.

Die US 5,853,413 zeigt eine Handgelenkarthrodeseplatte, die es ermöglicht, in mehrere Handwurzelknochen jeweils eine Knochenschraube einzubringen. Nachteilig ist, dass sich Knochenschrauben aus den Durchgangslöchern im Handwurzelknochenfixierabschnitt der Handgelenkarthrodeseplatte lösen können.

Die US 6,358,250 B1 zeigt keine Handgelenkarthrodeseplatte sondern eine Knochenplatte zur Behandlung der Fraktur des distalen Radius. Die bekannte Knochenplatte eignet sich nicht zur Fixierung oberhalb von Handwurzelknochen.

Der Erfindung liegt die Aufgabe zugrunde, eine alternative Handgelenkarthrodeseplatte vorzuschlagen, die eine sichere Versteifung des Handgelenks ermöglicht. Dabei sollte die Handgelenkarthrodeseplatte dem Operateur möglichst viele Freiheiten bei seinem chirurgischen Eingriff im Hinblick auf die Verankerungsmöglichkeiten bieten. Ferner besteht die Aufgabe darin, ein Sortiment, umfassend mindestens zwei wie zuvor beschrieben ausgebildete Handgelenkarthrodeseplatten vorzuschlagen.

Diese Aufgabe wird mit einer Handgelenkarthrodeseplatte mit den Merkmalen des Anspruchs 1 und hinsichtlich des Sortiments mit den Merkmalen des Anspruchs 13 gelöst. Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Der Erfindung liegt der Gedanke zugrunde, den Handwurzelknochenfixierabschnitt derart zu verbreitern, dass die Mittelpunkte mindestens zweier Durchgangslöcher zur Aufnahme jeweils mindestens einer Knochenschraube quer zur Längserstreckung der Handgelenkarthrodeseplatte beabstandet sind, derart, dass in mindestens zwei quer zur Längserstreckung der Handgelenkarthrodeseplatte, abschnittsweise oder vollständig, nebeneinander angeordnete Handwurzelknochen jeweils mindestens eine Knochenschraube einschraubbar ist. Anders ausgedrückt zeichnet sich eine nach dem Konzept der Erfindung ausgebildete Handgelenkarthrodeseplatte durch mindestens zwei, vorzugsweise mehr als zwei, quer zur Längserstreckung der Handgelenkarthrodeseplatte und ggf. auch in Richtung der Längserstreckung, zumindest abschnittsweise, versetzt angeordnete, vorzugsweise quer zur Längserstreckung der Handgelenkarthrodeseplatte sowie ggf. auch in Richtung der Längserstreckung voneinander beabstandete, Durchgangslöcher aus, die zur Aufnahme jeweils mindestens einer, vorzugsweise zur Aufnahme jeweils maximal einer, Knochenschraube ausgebildet sind, mit dem Ziel, eine Möglichkeit zu schaffen, die Handgelenkarthrodeseplatte, genauer den Handwurzelknochenfixierabschnitt, an mindestens zwei quer zur Längserstreckung, abschnittsweise oder vollständig, nebeneinander angeordneten Handwurzelknochen zu fixieren. Noch Anders ausgedrückt, sind die Durchgangslöcher derart angeordnet, dass oberhalb mindestens zweier, zumindest abschnittsweise, vorzugsweise vollständig, nebeneinander angeordneter Handwurzelknochen sich jeweils ein Durchgangsloch befindet. Noch anders ausgedrückt ist vorgesehen, dass die Durchgangslöcher im Handwurzelknochenfixierabschnitt derart quer zur Längserstreckung des Handgelenkarthrodeseplatte (1) beabstandet angeordnet sind, dass in mindestens zwei quer zur Längserstreckung der Handgelenkarthrodeseplatte, zumindest abschnittsweise, vorzugsweise vollständig, nebeneinander angeordnete Handwurzelknochen jeweils mindestens eine Knochenschraube einschraubbar ist. Ganz besonders bevorzugt umfasst der Handwurzelknochenfixierabschnitt eine Vielzahl von Durchgangslöchern, von denen mindestens zwei um mindestens einen Durchgangslochdurchmesser quer zur Längserstreckung der Handgelenksarthrodeseplatte betrachtet voneinander beabstandet sind. Ganz besonders bevorzugt ist eine Ausführungsform, bei der Knochenschrauben durch eine entsprechende seitliche Beabstandung der Mittelpunkte zweier Durchgangslöcher in mindestens zwei über mindestens einen Handwurzelknochen voneinander beabstandete Handwurzelknochen schraubbar sind. Um Weichteilstörungen und/oder Wundheilstörungen im Bereich der Mittelhandknochen (Ossametacarpalia) zu vermeiden, ist es besonders bevorzugt, wenn die, insbesondere aus einer Titanlegierung ausgebildete, Handgelenkarthrodeseplatte eine maximale Materialstärke von 3 mm, vorzugsweise von etwa 2,5mm oder darunter aufweist. Insgesamt zeichnet sich eine nach dem Konzept der Erfindung ausgebildete Handgelenkarthrodeseplatte durch die Möglichkeit einer Mehrfachverankerung im Bereich der Handwurzelknochen aus, mit der Folge, dass eine im Stand der Technik problematische Verankerung der Handgelenkarthrodeseplatte im dritten Mittelhandknochen besser unterstützt wird oder sogar, wie später noch erläutert werden wird, ganz entfallen kann. Bevorzugt ist der distale Fixierabschnitt zur Fixierung der Handgelenkarthrodeseplatte am Radius (Speiche) im Vergleich zum Stand der Technik verlängert, wodurch die Flexibilität in der Anwendung weiter erhöht wird. Insbesondere kann im Falle einer entsprechenden unterarmseitigen Verlängerung der Handgelenkarthrodeseplatte, in Abhängigkeit davon, wie viel Knochensubstanz im Bereich des Handgelenks oder der Handwurzelknochen noch zur Verfügung steht, die Handgelenkarthrodeseplatte flexibel angepasst werden.

In Weiterbildung der Erfindung ist mit Vorteil vorgesehen, dass die Durchgangslöcher im Handwurzelknochenfixierabschnitt derart verteilt angeordnet und in einer Anzahl vorgesehen sind, dass eine Fixierung der Handgelenkarthrodeseplatte bzw. des Handwurzelknochenfixierabschnitts in mindestens drei Handgelenkwurzelknochen mit jeweils mindestens einer Knochenschraube möglich ist. Auf diese Weise kann der Operateur auswählen, in welchem/welchen Handwurzelknochen eine Fixierung realisiert werden soll.

Besonders zweckmäßig ist eine Ausführungsform der Handgelenkarthrodeseplatte, bei der diese eine Vielzahl, d.h. mehr als drei, von Durchgangslöchern im Handwurzelfixierabschnitt aufweist. Dabei ist es besonders bevorzugt, wenn die Mittelpunkte von mindestens drei, vorzugsweise von mindestens vier, ganz besonders bevorzugt von fünf Durchgangslöchern quer zur Längserstreckung der Handgelenkarthrodeseplatte voneinander beabstandet sind. Zusätzlich oder alternativ ist es von Vorteil, wenn die Mittelpunkte von mindestens zwei, vorzugsweise von mindestens drei, ganz besonders bevorzugt von mindestens vier oder fünf Durchgangslöchern in Richtung der Längserstreckung der Handgelenksarthrodeseplatte voneinander beabstandet sind. Besonders bevorzugt wird eine flächige Matrix von Durchgangslöchern erhalten, die eine Fixierung der Handgelenkarthrodeseplatte an sämtlichen Handwurzelknochen ermöglicht.

Eine maximale Flexibilität für den Operateur wird erhalten, wenn im Handwurzelfixierabschnitt mindestens fünf voneinander beabstandete Durchgangslöcher vorgesehen sind. Besonders bevorzugt ist es, wenn mindestens acht, ganz besonders bevorzugt mindestens zehn oder mindestens dreizehn Durchgangslöcher im Handwurzelfixierabschnitt vorgesehen sind. Eine optimale Flexibilität für den Operateur wird erhalten, wenn der Handwurzelknochenfixierabschnitt der Handgelenkarthrodeseplatte vierzehn oder mehr Durchgangslöcher umfasst.

Um das Handgelenk in einer, zumindest weitgehend, natürlichen Entspannungsposition fixieren zu können, ist eine Ausführungsform von Vorteil, bei der der Handwurzelknochenfixierabschnitt ballig ausgeformt ist, sich also dorsal erhebt. Bevorzugt weist der Handwurzelknochenfixierabschnitt dabei, zumindest näherungsweise, eine Löffelform auf, um den anatomischen Gegebenheiten in möglichst vielen Fällen gerecht zu werden. Alternativ ist eine Ausführungsform realisierbar, bei der der Handwurzelknochenfixierabschnitt eben ausgeformt ist und bevorzugt, zumindest näherungsweise, in einer gemeinsamen Ebene mit dem distalen Fixierabschnitt liegt.

Besonders bevorzugt ist eine Ausführungsform der Handgelenkarthrodeseplatte, bei der der Handwurzelknochenfixierabschnitt sich in Bezug auf die Längserstreckung des proximalen Fixierabschnitts in zwei einander entgegengesetzte Querrichtungen erstreckt, wobei es weiter bevorzugt ist, wenn auf beiden sich in jeweils eine Querrichtung erstreckenden, also nicht mit dem proximalen Fixierabschnitt fluchtenden Flächenabschnitten des Handwurzelknochenfixierabschnitts jeweils mindestens eine, vorzugsweise jeweils mehrere Durchgangsöffnungen zur Aufnahme jeweils mindestens einer, vorzugsweise zur Aufnahme jeweils ausschließlich einer, Knochenschraube vorgesehen sind.

In Weiterbildung der Erfindung ist mit Vorteil vorgesehen, dass mindestens eine der Durchgangsöffnungen im Handwurzelknochenfixierabschnitt, vorzugsweise mehrere der im Handwurzelknochenfixierabschnitt angeordnete Durchgangsöffnungen, ganz besonders bevorzugt sämtliche Durchgangsöffnungen im Handwurzelknochenfixierabschnitt mit einem Innengewinde ausgestattet sind, das zur fixierenden Aufnahme einer Knochenschraube dient.

Besonders zweckmäßig ist es, wenn zur weiteren Erhöhung der Handhabungsflexibilität durch den Operateur im proximalen Fixierabschnitt zum Fixieren der Handgelenkarthrodeseplatte am Radius mindestens ein Langloch vorgesehen ist, das sich ganz besonders bevorzugt in Richtung der Längserstreckung der Handgelenkarthrodeseplatte erstreckt. Besonders bevorzugt ist es dabei, wenn sich das Langloch in Richtung auf den Handwurzelknochenfixierabschnitt zu verjüngt, wodurch der Einsatz unterschiedlich großer Knochenschrauben ermöglicht wird.

In Weiterbildung der Erfindung ist mit Vorteil vorgesehen, dass zusätzlich zu dem proximalen Fixierabschnitt ein sich distal von dem Handwurzelknochenfixierabschnitt weg erstreckender distaler Fixierabschnitt zum Fixieren der Handgelenkarthrodeseplatte an einem Mittelhandknochen und/oder an einem Fingerglied, insbesondere am ersten Fingerglied (phalanx proximalis) vorgesehen ist. Um auch hierbei eine maximale Flexibilität für den Operateur zu bieten, ist es bevorzugt, wenn im distalen Fixierabschnitt mindestens ein sich, insbesondere in Richtung der Längserstreckung des distalen Fixierabschnittes erstreckendes Langloch vorgesehen ist.

Um eine möglichst natürliche Fixierstellung zu realisieren, ist es besonders bevorzugt, wenn der distale Fixierabschnitt zum proximalen Fixierabschnitt unter einem Winkel geneigt ist. Bevorzugt beträgt der Neigungswinkel mindestens 5°, ganz besonders bevorzugt etwa 8°.

Idealerweise ist die Handgelenkarthrodeseplatte im Bereich ihres Handwurzelknochenfixierabschnitts verbiegbar, sodass der gewünschte Winkel auf einfache Weise einstellbar ist.

Aufgrund des im Vergleich zum Stand der Technik verbreiterten Handgelenkwurzelknochenfixierabschnittes ist auch eine Ausführungsform der Handgelenkarthrodeseplatte realisierbar, bei der bewusst auf das Vorsehen eines distalen Fixierabschnitts verzichtet wird, wodurch Entzündungsreaktionen im Bereich der Mittelhandknochen vollständig vermieden werden können.

Die Erfindung führt auch auf ein Sortiment, umfassend mindestens zwei wie zuvor beschriebene Handgelenkarthrodeseplatten, die sich in mindestens einem Formmerkmal unterscheiden. Dabei ist eine Ausführungsform des Sortiments besonders bevorzugt, bei der dieses mindestens eine Handgelenkarthrodeseplatte mit einem distalen Fixierabschnitt und mindestens eine Handgelenkarthrodeseplatte ohne distalen Fixierabschnitt aufweist.

Besonders bevorzugt ist eine Ausführungsform des Sortiments, bei der die distalen und/oder proximalen Fixierabschnitte zweier Handgelenkarthrodeseplatten des Sortiments seitlich relativ zueinander versetzt angeordnet sind, um eine Fixierung an unterschiedlichen Querpositionen des Radius bzw. an eine Fixierung an unterschiedlichen Mittelhandknochen zu ermöglichen.

Weitere Vorteile, Merkmale und Einzelheiten der Erfindung ergeben sich aus der nachfolgenden Beschreibung bevorzugter Ausführungsbeispiele sowie anhand der Zeichnungen. In diesen sind Abmessungen von bevorzugten Ausführungsformen angegeben. Die Abmessungen sowie sich aus diesen ergebende Abmessungsverhältnisse sollen als erfindungswesentlich offenbart gelten und beanspruchbar sein. Die Abmessungen und Abmessungsverhältnisse in den Zeichnungen sollen den Schutzumfang der in den Ansprüchen zum Ausdruck kommenden Erfindung jedoch nicht einschränken.

Es zeigen:
- Fig. 1a bis 1f: unterschiedliche Ansichten und Schnittdarstellungen eines ersten Ausführungsbeispiels der dorsalen Handgelenkarthrodeseplatte,
- Fig. 2: eine alternative Ausführungsform einer Handgelenkarthrodeseplatte zur Fixierung des linken Handgelenks,
- Fig. 3a und 3b: unterschiedliche Ansichten einer Ausführungsform einer Handgelenkarthrodeseplatte zur Fixierung eines rechten Handgelenks,
- Fig.4: eine Ausführungsform einer Handgelenkarthrodeseplatte ohne distalen Fixierabschnitt zum Fixieren eines linken Handgelenks,
- Fig. 5a und 5b: unterschiedliche Ansichten einer Handgelenkarthrodeseplatte ohne distalen Fixierabschnitt zur Fixierung eines rechten Handgelenks,
- Fig. 6a bis 6c: unterschiedliche, teilweise geschnittene Ansichten einer Ausführungsform einer Handgelenkarthrodeseplatte mit flachem Handwurzelknochenfixierabschnitt
- Fig. 7a und 7b: eine alternative Ausführungsform einer Handgelenkarthrodeseplatte mit flachem Handwurzelknochenfixierabschnitt, jedoch ohne distalen Fixierabschnitt,
- Fig.8: eine Ausführungsform einer Handgelenkarthrodeseplatte mit flachem (ebenem) Handwurzelknochenfixierabschnitt sowie mit einem distalen Fixierabschnitt zur Fixierung eines linken Handgelenks,
- Fig. 9a und 9b: eine Ausführungsform einer Handgelenkarthrodeseplatte zur Fixierung eines rechten Handgelenks,
- Fig. 10: eine Ausführungsform einer Handgelenkarthrodeseplatte mit ebenem Handwurzelknochenfixierabschnitt, jedoch ohne distalen Fixierabschnitt zur Fixierung eines linken Handgelenks,
- Fig. 11a und 11b: unterschiedliche Ansichten einer Handgelenkarthrodeseplatte ohne distalen Fixierabschnitt zur Fixierung eines rechten Handgelenks,
- Fig. 12: die in den Fig. 6a bis 6b gezeigte Arthrodeseplatte, teilweise fixiert an einem linken Handgelenk, und
- Fig. 13: die in den Fig. 7a und 7b gezeigte Handgelenkarthrodeseplatte, teilweise fixiert an einem linken Handgelenk.

In den Figuren sind gleiche Elemente und Elemente mit der gleichen Funktion mit den gleichen Bezugszeichen gekennzeichnet.

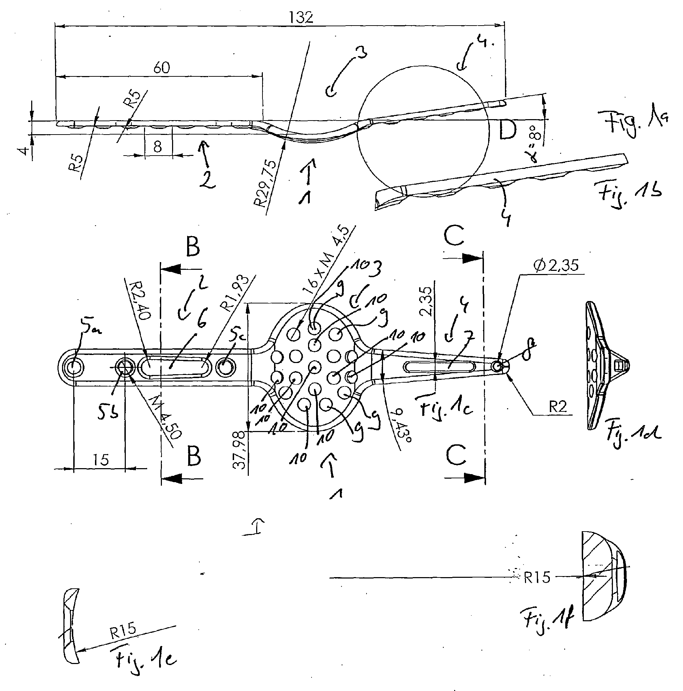

In den Fig. 1a bis 1f ist, teilweise in geschnittenen Darstellungen, eine Handgelenkarthrodeseplatte 1 gezeigt. Mit dieser kann alternativ ein linkes oder ein rechtes Handgelenk fixiert werden. Hierzu ist die Handgelenkarthrodeseplatte symmetrisch zu einer gedachten Längsmittelachse ausgebildet. Die Handgelenkarthrodeseplatte 1 ist unterteilbar in drei Abschnitte, nämlich einen in der Zeichnungsebene links angeordneten proximalen Fixierabschnitt 2 zum Festlegen der Handgelenkarthrodeseplatte 1 am Radius, einem mittleren Handwurzelknochenfixierabschnitt 3 zum Festlegen der Handgelenkarthrodeseplatte 1 in mindestens zwei Handwurzelknochen und einem distalen Fixierabschnitt 4 zum Fixieren der Handgelenkarthrodeseplatte 1 an einem Mittelhandknochen und/oder an einem Fingergliedknochen. Die Fixierabschnitte 2, 4 sind als langgestreckte Metallabschnitte von geringer Breitenerstreckung ausgeformt, wobei die Breitenerstreckung des Handwurzelknochenfixierabschnittes 3 größer ist als dessen Längserstreckung.

Um eine optimale Anlage des proximalen Fixierabschnittes 2 am Radius zu ermöglichen, ist der Fixierabschnitt 2, wie sich aus Fig. 1e ergibt, quer zu dessen Längserstreckung auf der Unterseite gewölbt ausgeführt. Im proximalen Fixierabschnitt 2 befinden sich drei in Längserstreckung der Handgelenkarthrodeseplatte 1 voneinander beabstandete Fixerlöcher 5a, 5b, 5c mit jeweils einem Innengewinde zur jeweiligen Aufnahme einer einzigen Knochenschraube. Wie sich aus Fig. 1c weiter ergibt, befindet sich mittig zwischen dem mittleren Fixierloch 5b und dem in der Zeichnungsebene rechten Fixierloch 5c ein sich in Richtung der Längserstreckung des proximalen Fixierabschnittes 2 erstreckendes Langloch 6, dessen Breitenerstreckung, d.h. dessen Erstreckung quer zur Längserstreckung des proximalen Fixierabschnittes 2 in Richtung hin zum Handwurzelknochenfixierabschnitt 3 abnimmt, und zwar von einem Radius R 2,4 hin zu einem Radius R 1,93.

Wie sich insbesondere aus Fig. 1a ergibt, ist die im fixierten Zustand obere, in der Zeichnungsebene untere Seite der Handgelenkarthrodeseplatte 1 wellenförmig strukturiert, um ein erleichtertes, definiertes Verbiegen der Handgelenkarthrodeseplatte 1 zur Anpassung an die anatomischen Gegebenheiten zu ermöglichen.

Während die Breitenerstreckung des proximalen Fixierabschnittes 2 im Wesentlichen über seine Längserstreckung konstant bleibt, nimmt die Breitenerstreckung des distalen Fixierabschnittes 4 hin zu dessen freien Ende ab. Im distalen Fixierabschnitt 4 befindet sich ein Langloch 7 mit konstanter Breitenerstreckung. Im Bereich des freien Endes des distalen Fixierabschnittes 4 befindet sich ein Fixierloch 8 zur Aufnahme einer Knochenschraube.

Wie sich aus der Detailvergrößerung gemäß Fig. 1b, die einen Abschnitt des distalen Fixierabschnittes 4 zeigt, ergibt, ist auch die im Fixierzustand obere Seite des distalen Fixierabschnittes 4 wellenförmig ausgeformt, wobei die Wellenamplituden, analog zum proximalen Fixierabschnitt 2 abgeflacht ausgeformt sind.

Aus Fig. 1a ergibt sich, dass der langgestreckte distale Fixierabschnitt unter einem Winkel α von in diesem Ausführungsbeispiel 8° zur Längserstreckung des proximalen Fixierabschnittes 2 geneigt ist.

Der mittig zwischen den Fixierabschnitten 2, 4 angeordnete Handwurzelknochenfixierabschnitt 3 ist, wie sich aus Fig. 1a ergibt, ballig (konvex) ausgeformt und erstreckt sich in Richtung von dem Handgelenk weg, d.h. in dorsale Richtung. Aus einer Zusammenschau der Fig. 1a, 1c und 1d ergibt sich, dass der Handwurzelknochenfixierabschnitt 3 löffelartig gekrümmt ist und im Wesentlichen in der Draufsicht gemäß Fig. 1c oval konturiert ist.

In der Flächenerstreckung des Handwurzelknochenfixierabschnittes 3 sind eine Vielzahl, hier achtzehn, Durchgangslöcher 9 eingebracht, die jeweils die Aufnahme einer Knochenschraube ermöglichen. Zu erkennen ist, dass die Mittelachsen der Durchgangslöcher 9 je nach Relativposition zueinander geneigt sind. Dies ist darauf zurückzuführen, dass die Durchgangslöcher 9 sich im Wesentlichen senkrecht zur in Längsrichtung gekrümmten Oberfläche des Handwurzelknochenfixierabschnittes 3 erstrecken.

Wie sich aus Fig. 1c und Fig. 1d ergibt, sind mindestens fünf Durchgangslöcher 9 vorgesehen, deren Mittelpunkte 10 quer zur Längserstreckung der Handgelenkarthrodeseplatte beabstandet sind. Ebenso sind mindestens fünf Durchgangslöcher 9 vorgesehen, deren Mittelpunkte 10 in Richtung der Längserstreckung der Handgelenkarthrodeseplatte 1 beabstandet sind. Insgesamt sind die Durchgangslöcher 9 derart verteilt angeordnet, dass in jedem der insgesamt sieben Handwurzelknochen der menschlichen Hand jeweils eine Knochenschraube eindrehbar ist. Wie sich weiterhin aus Fig. 1c ergibt, sind zwischen zwei in Richtung der Längserstreckung der Handgelenkarthrodeseplatte 1 maximal beabstandeten Durchgangslöcher 9 mindestens drei Durchgangslöcher 9 angeordnet. Zwischen zwei maximal quer zur Längserstreckung der Handgelenkarthrodeseplatte 1 beabstandeten Durchgangslöchern befinden sich ebenfalls mindestens drei weitere Durchgangslöcher 9.

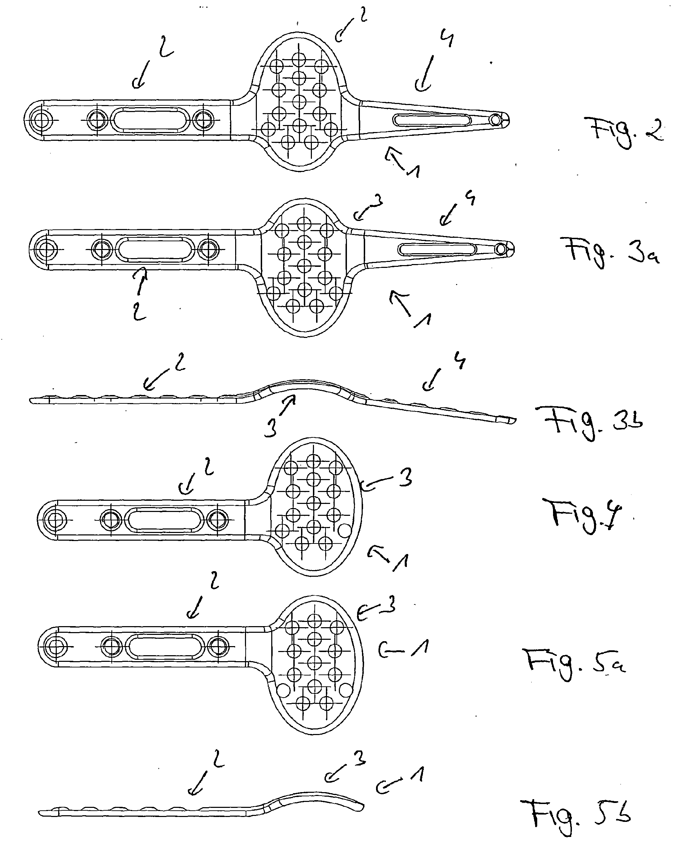

In Fig. 2 ist eine Handgelenkarthrodeseplatte 1 zur Fixierung eines linken Handgelenks gezeigt. Im Gegensatz zu dem Ausführungsbeispiel gemäß den Fig. 1a bis 1f ist diese Handgelenkarthrodeseplatte 1 nicht spiegelsymmetrisch zu einer Längsmittelachse angeordnet. Vielmehr sind sowohl der proximale Fixierabschnitt 2 als auch der distale Fixierabschnitt 4 seitlich zu einer gedachten Längsmittelachse versetzt angeordnet, wobei auch bei dem Ausführungsbeispiel gemäß Fig. 2 die beiden Fixierabschnitte 2, 4 in Richtung der Längserstreckung der Handgelenkarthrodeseplatte 1 unter Einschluss eines Winkels fluchtend angeordnet sind.

In den Fig. 3a und 3b ist eine zur Fixierung eines rechten Handgelenks ausgebildete Handgelenkarthrodeseplatte 1 mit einem proximalen, einem distalen sowie einem Handwurzelknochenfixierabschnitt 2, 4, 3 gezeigt. Bei sämtlichen Handgelenkarthrodesenplatten 1 gemäß den Fig. 2 bis 3b ist zu erkennen, dass der Handwurzelknochenfixierabschnitt 3 in Richtung von dem Handgelenk weg gewölbt, d.h. ballig, in der Art eines Löffels ausgeformt ist.

In den Fig. 4 bis 5b sind alternative Handgelenkarthrodeseplatten 1 gezeigt, die im Unterschied zu den zuvor beschriebenen Ausführungsbeispielen bewusst auf einen distalen Fixierabschnitt verzichten. Die Handgelenkarthrodeseplatten 1 gemäß den Fig. 4 bis 5b umfassen ausschließlich einen proximalen Fixierabschnitt 2 sowie einen daran anschließenden Handwurzelknochenfixierabschnitt 3, wobei der proximale Fixierabschnitt 2 sowie der Handwurzelknochenfixierabschnitt 3 im Wesentlichen analog zu den Ausführungsbeispielen gemäß den Fig. 2 bis 3b ausgebildet sind. Die in Fig. 4 gezeigte Ausführungsform mit einem in der Zeichnungsebene relativ zu einer gedachten, mittig durch den Handwurzelknochenfixierabschnitt 3 verlaufenden Längsmittelachse versetzten proximalen Fixierabschnitt 2 eignet sich zur Fixierung eines linken Handgelenks, wohingegen die in Fig. 5a und 5b gezeigte Ausführungsform eine Handgelenkarthrodeseplatte 1 zeigt, welche zum Fixieren eines rechten Handgelenks geeignet ist.

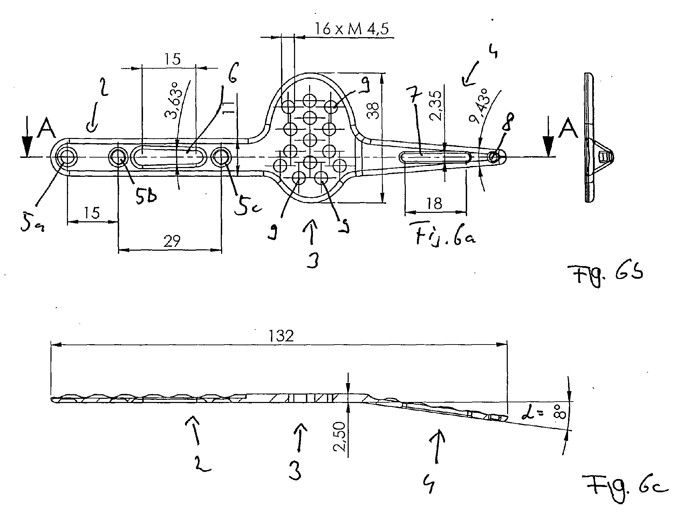

In den Fig. 6a bis 6c ist eine Ausführungsform einer Handgelenkarthrodeseplatte 1 zu entnehmen, die zum Fixieren eines linken Handgelenks ausgebildet ist. Im Gegensatz zu sämtlichen zuvor beschriebenen Handgelenkarthrodeseplatten 1, ist der mittige Handwurzelknochenfixierabschnitt 3 eben ausgeformt, sodass die Mittelachsen der Vielzahl von Durchgangslöchern 9 parallel zueinander orientiert sind. Im proximalen Fixierabschnitt 2 befinden sich insgesamt drei voneinander beabstandete Fixierlöcher 5a, 5b, 5c zur Aufnahme jeweils einer Fixierschraube, wobei zwischen den Fixierlöchern 5b und 5c ein sich verjüngendes Langloch 6 angeordnet ist. Im Gegensatz dazu verjüngt sich das Langloch 7 im distalen Fixierabschnitt 4 nicht. Endseitig ist im distalen Fixierabschnitt 4 ein Fixierloch 8 vorgesehen, dessen Innengewinde einen kleineren Durchmesser aufweist, als die Innengewinde der Fixierlöcher 5a, 5b, 5c.

Wie sich aus den Fig. 6b und 6c ergibt, ist der distale Fixierabschnitt 4 zu dem proximalen Fixierabschnitt 2 sowie zu dem ebenen Handwurzelknochenfixierabschnitt 3 unter einem Winkel α von 8° geneigt.

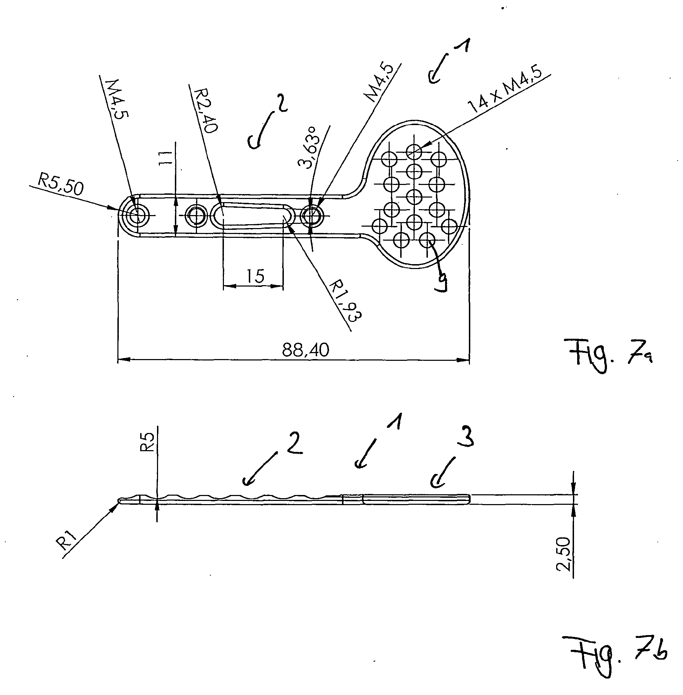

In Fig. 7a und 7b ist ein Ausführungsbeispiel einer Handgelenkarthrodeseplatte 1 gezeigt, bei der bewusst auf das Vorsehen eines distalen Fixierabschnittes verzichtet wurde. Die Handgelenkarthrodeseplatte 1 umfasst einen proximalen Fixierabschnitt 2, der analog zu den vorangehenden Ausführungsbeispielen ausgeformt ist. Der ebene, oval konturierte Handwurzelknochenfixierabschnitt 3 umfasst insgesamt vierzehn Durchgangslöcher 9, wobei die Durchgangslöcher 9 im Wesentlichen in sich quer zur Längserstreckung der Handgelenkarthrodeseplatte 1 erstreckenden Reihen von jeweils vier oder fünf Durchgangslöchern angeordnet sind.

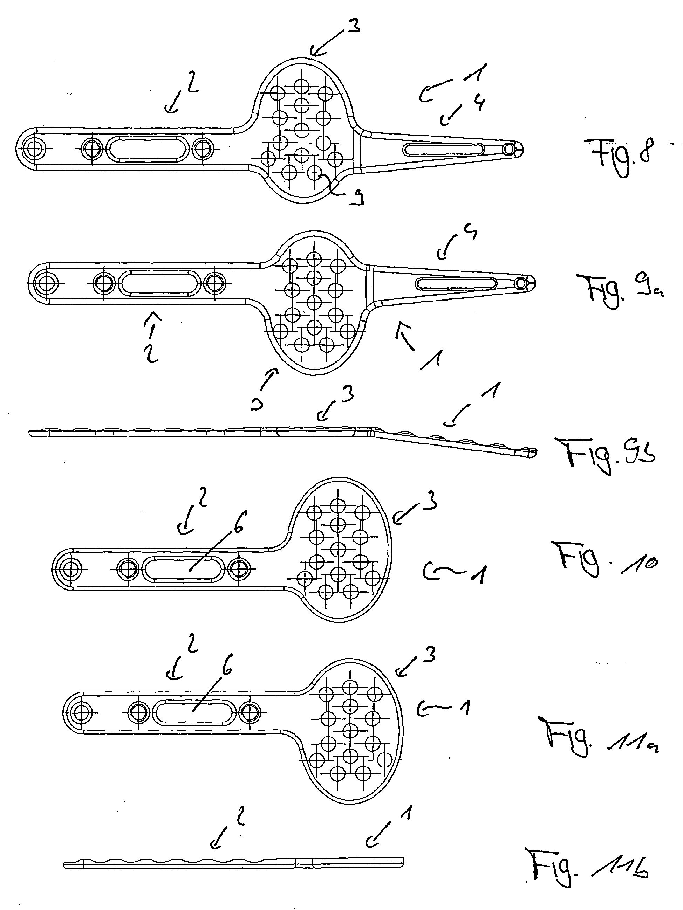

In Fig. 8 ist das Ausführungsbeispiel gemäß Fig. 6a nochmals ohne Bemaßungen dargestellt. Die Handgelenkarthrodeseplatte 1 dient zur Fixierung eines linken Handgelenks und weist hierzu zwei, in der Zeichnungsebene nach unten relativ zu einer gedachten, mittig durch den Handwurzelknochenfixierabschnitt 3 verlaufenden, Längsmittelachse versetzte Fixierabschnitte 2, 4 auf, die zwischen sich den Handwurzelknochenfixierabschnitt 3, der in dem gezeigten Ausführungsbeispiel eben ausgeformt ist, zwischen sich aufnehmen.

In den Fig. 9a und 9b ist eine zur Fixierung eines rechten Handgelenks ausgebildete Handgelenkarthrodeseplatte 1 gezeigt. Der proximale und der distale Fixierabschnitt 2, 4 sind in der Zeichnungsebene nach oben relativ zu einer gedachten, mittig durch den Handwurzelknochenfixierabschnitt 3 verlaufenden, Längsmittelachse versetzt angeordnet. Fig. 9b ist die flache Ausführung des Handwurzelfixierabschnittes 3 zu entnehmen.

Fig. 10 bis 11b zeigen Ausführungsformen von Handgelenkarthrodeseplatten 1 jeweils ohne distalen Fixierabschnitt 4, lediglich mit proximalem Fixierabschnitt 2, wobei das Langloch 6 im proximalen Fixierabschnitt 2 analog zu den Ausführungsbeispielen gemäß den Fig. 8 bis 9b, im Gegensatz zu den Ausführungsbeispielen 1 bis 7b sich nicht verjüngend ausgebildet ist. Die in Fig. 10 gezeigte Handgelenkarthrodeseplatte 1 eignet sich zur Fixierung eines linken Handgelenks, wohingegen die in den Fig. 11a bis 11b gezeigte Handgelenkarthrodeseplatte 1 zur Fixierung eines rechten Handgelenkes ausgebildet ist. Fig. 11b ist die flache Ausformung des Handwurzelknochenfixierabschnittes 3 zu entnehmen.

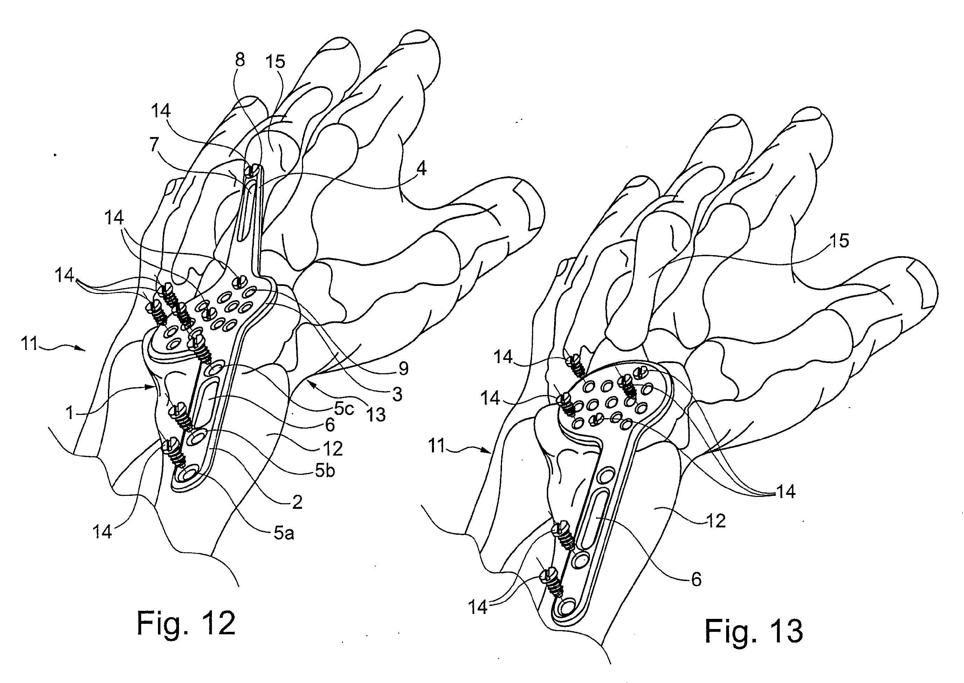

In Fig. 12 ist eine Handgelenkarthrodeseplatte 1 mit seitlich versetztem, proximalen und distalen Fixierabschnitt 2, 4 gezeigt. Der Handwurzelknochenfixierabschnitt 3 befindet sich oberhalb der Handwurzelknochen 11, die sich distal von dem Radius 12 (Speiche) und dem Handgelenk 13, welches wiederum zwischen dem Radius 12 und dem Handwurzelknochen 11 ausgebildet ist, befinden. In den Fixierlöchern 5a, 5b, 5c soll jeweils eine Knochenschraube 14 aufgenommen werden. Des Weiteren sind im gezeigten Ausführungsbeispiel insgesamt fünf Knochenschrauben 14 zur Aufnahme in unterschiedlichen Durchgangsöffnungen 9 im Handwurzelknochenfixierabschnitt 3 vorgesehen. Der distale Fixierabschnitt 4 ist mit Hilfe einer einzigen Knochenschraube 14 im Fixierloch 8 am dritten Mittelhandknochen 15 fixiert.

In Fig. 13 ist eine teilweise an einem linken Arm fixierte Handgelenkarthrodeseplatte 1 gezeigt. Diese umfasst keinen distalen Fixierabschnitt und wird mit Hilfe von zwei Knochenschrauben 14 am Radius 12 fixiert. Der Handwurzelknochenfixierabschnitt 3 kann mit maximal vierzehn verteilt angeordneten Knochenschrauben 14 in mehr als drei Handwurzelknochen 11 fixiert werden. Zusätzlich oder alternativ zu den in den Fig. 12 und 13 gezeigten Fixiermöglichkeiten des proximalen und distalen Fixierabschnittes 2, 4 ist es möglich, den proximalen oder distalen Fixierabschnitt 2, 4 durch Anordnung mindestens einer Knochenschraube in einem Langloch 6, 7 am Radius 12 bzw. am Mittelhandknochen 15 zu fixieren. Eine Fixiermöglichkeit an dem Mittelhandknochen 15 ist bei dem Ausführungsbeispiel gemäß Fig. 13 bewusst nicht gegeben und nicht notwendig.

## Patentansprüche

1. Handgelenkarthrodeseplatte, umfassend einen proximalen Fixierabschnitt (2) zur Fixierung der Handgelenkarthrodeseplatte (1) am Radius (12) sowie einen, mehrere Durchgangslöcher (9) zur Aufnahme jeweils mindestens einer Knochenschraube (14) aufweisenden, Handwurzelknochenfixierabschnitt (3) zum Fixieren der Handgelenkarthrodeseplatte (1) an Handwurzelknochen (11), wobei die Durchgangslöcher (9) im Handwurzelknochenfixierabschnitt (3) derart angeordnet sind, dass oberhalb mindestens zweier Handwurzelknochen sich jeweils eines der Durchgangslöcher (8) befindet,
**dadurch gekennzeichnet,**
**dass** zumindest eines der Durchgangslöcher (9) im Handwurzelknochenfixierabschnitt (3) ein Innengewinde aufweist.

2. Handgelenkarthrodeseplatte nach Anspruch 1, **dadurch gekennzeichnet, dass** die Durchgangslöcher (9) derart verteilt angeordnet und in einer Anzahl vorgesehen sind, dass in mindestens drei, vorzugsweise in jeden, Handwurzelknochen (11) mindestens eine Knochenschraube (14) einbringar ist.

3. Handgelenkarthrodeseplatte nach einem der vorhergehenden Ansprüche, **dadurch gekennzeichnet,**
**dass** im Handwurzel-knochenfixierabschnitt (3) die Mittelpunkte (10) von mindestens drei, vorzugsweise von mindestens vier, Durchgangslöchern (9) quer zur Längserstreckung der Handgelenkarthrodeseplatte (1) nebeneinander und/oder die Mittelpunkte (10) von mindestens zwei, vorzugsweise von mindestens drei, bevorzugt von mindestens vier, Durchgangslöchern (9) in Richtung der Längserstreckung der Handgelenkarthrodeseplatte (1) hintereinander angeordnet sind.

4. Handgelenkarthrodeseplatte nach einem der vorhergehenden Ansprüche, **dadurch gekennzeichnet,**
**dass** mindestens fünf, vorzugsweise mindestens acht, bevorzugt mindestens zehn, besonders bevorzugt mindestens zwölf, ganz besonders bevorzugt mindestens dreizehn Durchgangslöcher (9) im Handwurzelknochenfixierabschnitt (3) vorgesehen sind.

5. Handgelenkarthrodeseplatte nach einem der vorhergehenden Ansprüche, **dadurch gekennzeichnet,**
**dass** der Handwurzelknochenfixierabschnitt (3) ballig, vorzugsweise löffelförmig, oder eben ausgeformt ist.

6. Handgelenkarthrodeseplatte nach einem der vorhergehenden Ansprüche, **dadurch gekennzeichnet,**
**dass** mehrere, insbesondere sämtliche Durchgangslöcher (9) im Handwurzelknochenfixierabschnitt (3) ein Innengewinde aufweisen.

7. Handgelenkarthrodeseplatte nach einem der vorhergehenden Ansprüche, **dadurch gekennzeichnet,**
**dass** der Handwurzelknochenfixierabschnitt (3) den proximalen Fixierabschnitt (2) quer zu dessen Längserstreckung zweiseitig überragt.

8. Handgelenkarthrodeseplatte nach einem der vorhergehenden Ansprüche, **dadurch gekennzeichnet,**
**dass** in dem proximalen Fixierabschnitt (2) mindestens ein, vorzugsweise sich in Richtung des Handwurzelknochenfixierabschnitts (3) verjüngendes, Langloch (6) vorgesehen ist.

9. Handgelenkarthrodeseplatte nach einem der vorhergehenden Ansprüche, **dadurch gekennzeichnet,**
**dass** ein, sich vorzugsweise in eine distale Richtung verjüngter, distaler Fixierabschnitt (4) zum Fixieren der Handgelenkarthrodeseplatte (1) an einem Mittelhandknochen (15) und/oder an einem Fingerglied vorgesehen ist.

10. Handgelenkarthrodeseplatte nach Anspruch 9, **dadurch gekennzeichnet, dass** in dem distalen Fixierabschnitt (4) mindestens ein Langloch (7) vorgesehen ist.

11. Handgelenkarthrodeseplatte nach eine der Ansprüche 9 oder 10,
**dadurch gekennzeichnet,**
**dass** der distale Fixierabschnitt (4) zum proximalen Fixierabschnitt (2) unter einem Winkel (?), vorzugsweise von mehr als 5°, geneigt ist.

12. Handgelenkarthrodeseplatte nach einem der Ansprüche 1 bis 8,
**dadurch gekennzeichnet,**
**dass** die Handgelenkarthrodeseplatte (1) keinen an den Handwurzelknochenfixierabschnitt (3) angrenzenden, distalen Fixierabschnitt (4) aufweist.

13. Sortiment, umfassend mindestens zwei Handgelenkarthrodeseplatten (1) nach einem der vorhergehenden Ansprüche, wobei der distale und/oder der proximale Fixierabschnitt (2, 4) von mindestens zwei, vorzugsweise von drei, der Handgelenkarthrodeseplatten (1) des Sortiments an einer unterschiedlichen Relativposition quer zur Längserstreckung der jeweiligen Handgelenkarthrodeseplatte (1) angeordnet ist.

14. Sortiment nach Anspruch 13, **dadurch gekennzeichnet,**
**dass** mindestens eine der Handgelenkarthrodeseplatten (1) keinen distalen Fixierabschnitt (4) aufweist.
